# EUROPÄISCHE PATENTSCHRIFT

(11) **EP 0 713 673 B1**
(45) Veröffentlichungstag und Bekanntmachung des Hinweises auf die Patenterteilung: **12.02.2003**
(21) Anmeldenummer: 95117725.2
(22) Anmeldetag: 09.11.1995
(51) Int. Cl.: A61B 5/087

(54) **Messkopf**
Measuring head
Tête de mesure

(30) Priorität: 10.11.1994 DE 4440161
(43) Veröffentlichungstag der Anmeldung: 29.05.1996
(73) Patentinhaber: Müller & Sebastiani Elektronik-GmbH, 81377 München (DE)
(72) Erfinder: Müller, Peter, D-80798 München (DE); Sebastiani, Oscar, D-80469 München (DE)
(74) Vertreter: Manitz, Finsterwald & Partner

(56) Entgegenhaltungen:
- EP-A- 0 035 680
- EP-A- 0 295 575
- DE-A- 3 529 367
- DE-U- 9 113 996
- US-A- 3 626 755
- US-A- 4 297 900
- US-A- 5 060 655

## Beschreibung

Die Erfindung betrifft einen Messkopf zur Bestimmung eines Gasvolumenstroms mit einem insbesondere an einem Handgriff angeordneten Messrohr, das mit einem sich im wesentlichen senkrecht zu seiner Achse erstreckenden feinmaschigen Drahtnetz versehen ist, an dessen beiden Seiten eine Druckerfassung erfolgt.

Derartige Messköpfe sind grundsätzlich bekannt und werden beispielsweise für Messungen in Zusammenhang mit Lungenfunktionsanalysen eingesetzt.

Die US 5,060,655 offenbart einen Messkopf zur Bestimmung eines Gasvolumenstroms, der zwei Teilrohre sowie zwei Ringe aufweist, in denen Bohrungen vorgesehen sind, die mit Öffnungen im Gehäuse korrespondieren.

Aus der DE 35 29 367 A1 ist ein Messkopf mit einem Messrohr bekannt, das mit einem Druckaufnehmer über im Messrohr ausgebildete Ringkanäle verbunden ist.

Es ist eine Aufgabe der Erfindung, einen verbesserten Messkopf zu schaffen, der vielseitig verwendbar und einfach aufgebaut ist.

Diese Aufgabe wird erfindungsgemäß im wesentlichen dadurch gelöst, dass das Messrohr aus zwei im wesentlichen koaxial ausgerichteten, mit dem Messkopf lösbar verbundenen Teilrohren besteht, zwischen denen das Drahtnetz gehalten ist, und beiderseits des Drahtnetzes sich über den Umfang des Messrohrs erstreckende Ringkanäle ausgebildet sind, die einerseits mit dem Innenraum des Messrohrs und andererseits mit zumindest einem Drucksensor in Verbindung stehen, wobei die Ringkanäle zwischen den Außenwänden oder den Stirnseiten der Teilrohre und einer im Messkopf vorgesehenen Aufnahme für die Teilrohre ausgebildet sind.

Bei dem erfindungsgemäßen Messkopf können auf besonders einfache Weise durch Lösen der Verbindungen zwischen dem Messkopf und den Teilrohren die einzelnen Messkopf-Bauteile gereinigt bzw. ausgewechselt werden.

Dies ist besonders im Hinblick auf das Drahtnetz von Vorteil, dessen Verschmutzung durch den Niederschlag von Feuchtigkeit in der Atemluft nicht zu vermeiden ist und das aufgrund des vorteilhaften Messkopfbaubaus auf einfache Weise entnommen werden kann.

Der erfindungsgemäße Messkopf kann außer für Lungenfunktionsanalysen auch für Messungen in Zusammenhang mit der Rhinomanometrie (Messung des Nasenwiderstandes) verwendet werden.

Weitere Ausführungsformen der Erfindung sind in den Unteransprüchen angegeben.

Die Erfindung wird im folgenden beispielhaft anhand der Zeichnung beschrieben, deren einzige Figur eine mögliche Ausführungsform eines erfindungsgemäßen Messkopfs zeigt.

Der dargestellte Messkopf kann beispielsweise durch Kopplung mit einem entsprechenden Mundstück zur Bestimmung der Funktionsparameter bei der Durchführung einer Lungenfunktionsanalyse verwendet werden.

Der Messkopf besteht im wesentlichen aus einem an einem Handgriff 28 angeordneten Messrohr 29, 30, das mit einem sich im wesentlichen senkrecht zu seiner Achse erstreckenden feinmaschigen Drahtnetz 31 versehen ist.

Das Messrohr 29, 30 ist dabei in einer ringförmigen Aufnahme 32 gehalten, welche am oberen Ende des Handgriffs 28 angebracht ist. Der Innendurchmesser der ringförmigen Aufnahme 32 entspricht im wesentlichen dem Außendurchmesser desjenigen Bereichs des Messrohres 29, 30, welcher in der ringförmigen Aufnahme 32 gehalten ist.

Das Messrohr 29, 30 besteht aus zwei koaxial ausgerichteten Teilrohren 29 und 30, die von beiden Seiten der Aufnahme 32 in diese derart einschraubbar sind, dass sie sich stirnseitig gegenüberliegen.

Die Teilrohre 29, 30, zwischen denen das Drahtnetz 31 gehalten ist, sind jeweils an ihrem dem Drahtnetz 31 abgewandten Endbereich mit einem düsenartigen, sich mit zunehmendem Abstand vom Drahtnetz stetig verjüngenden trompetenförmigen Ansatz versehen. Dieser düsenartige Ansatz erstreckt sich jedoch nicht bis direkt an das Drahtnetz 31, da in jedem Teilrohr 29, 30 zwischen dem düsenartigen Ansatz und dem Drahtnetz 31 noch ein im wesentlichen zylindrisch ausgebildeter Bereich vorgesehen ist.

Durch die beschriebene Formgebung der Teilrohre 29, 30 wird einerseits erreicht, daß die Strömungsgeschwindigkeit des durch das Messrohr 29, 30 strömenden Gases im Bereich des Drahtnetzes aufgrund des dort vergrößerten Messrohr-Durchmessers reduziert wird. Andererseits wird durch die düsenartige Form des Messrohres das Problem der unterschiedlichen Durchmesser des Drahtnetzes 31 und eines mit dem Messrohr 29, 30 gekoppelten Mundstücks (nicht dargestellt) gelöst, da sich diesbezüglich durch den sich in Richtung des Drahtnetzes 31 stetig vergrößernden Durchmesser des Messrohres 29, 30 eine entsprechende Adaption ergibt. Da auf diese Weise ein stetiger Übergang von dem vergleichsweise kleinen Durchmesser des Mundstücks bzw. des dem Drahtnetz 31 abgewandten Endes des jeweiligen Teilrohres 29, 30 zu dem vergleichsweise großen Durchmesser des Drahtnetzes 31 erreicht wird, stellt sich im Messrohr 29, 30 eine weitgehend wirbelfreie Strömung ein, welche im Bereich des Drahtnetzes 31 im wesentlichen laminar verläuft.

Beiderseits des Drahtnetzes 31 ist jeweils ein Schutzelement 33 vorgesehen, welches in Strömungsrichtung verlaufende Durchbrechungen aufweist. Die Schutzelemente 33 erstrecken sich im wesentlichen parallel zum Drahtnetz 31 und weisen einen Durchmesser auf, der dem Innendurchmesser der Teilrohre 29, 30 in demjenigen Bereich entspricht, in dem die Schutzelemente 33 vorgesehen sind. Der Abstand zwischen den Schutzelementen 33 und dem Drahtnetz 31 ist relativ klein, insbesondere beträgt er weniger als 0,5 cm.

Die bevorzugte, in der Figur dargestellte Ausführungsform dieser Schutzelemente zeichnet sich dadurch aus, dass sie sich in Strömungsrichtung über einen Bereich von ungefähr 0,3 bis 0,8 cm erstreckt und eine Wabenstruktur aufweist, wobei die einzelnen Waben in Strömungsrichtung ausgerichtet sind. Die einzelnen Waben können dabei einen Durchmesser von ungefähr 3 bis 3,5 mm besitzen.

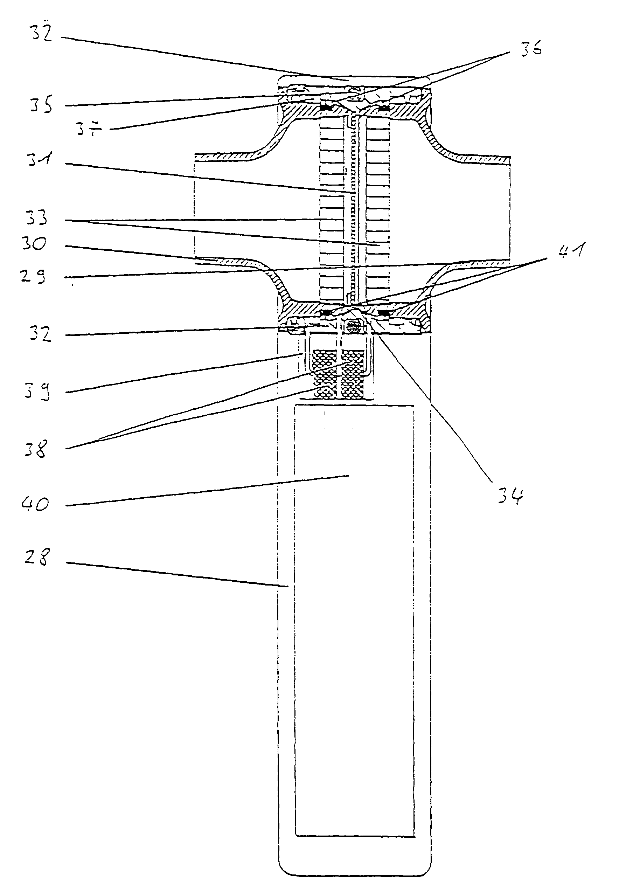

Durch die Vorsehung derartiger, eine Wabenstruktur aufweisender Schutzelemente 33 wird über die Schutzfunktion bezüglich des Drahtnetzes 31 hinaus noch erreicht, dass sich im Bereich des Drahtnetzes 31 eine sehr gleichmäßige und laminare Strömung einstellt, da die einzelnen, in Strömungsrichtung ausgerichteten Waben das durch sie strömende Gas ohne die Bildung von Wirbeln gleichmäßig und senkrecht zum Drahtnetz 31 durch dieses hindurch führen. Dies gilt bei Vorsehung von zwei Schutzelementen 33 für beide Strömungsrichtungen, so dass der in Figur 8 dargestellte Messkopf in Verbindung mit einem Mundstück beispielsweise auch zur gleichzeitigen Erfassung der inspiratorischen und der exspiratorischen Phase der Atmung eines Patienten eingesetzt werden kann.

Die Schutzelemente 33 sind bevorzugt in jedem Teilrohr 29, 30 lösbar, insbesondere mittels einer Schraubverbindung befestigt. Dies vereinfacht die Zerlegung des Messkopfes beispielsweise zum Zwecke der Reinigung, der Reparatur oder des Austauschs einzelner Teile.

Die ringförmige Aufnahme 32 für die Teilrohre 29, 30 weist in ihrem axial mittleren Bereich einen ringförmigen, radial nach innen vorspringenden Flansch 34 auf, dessen radial innenliegenden Stirnseiten eine Kreislinie beschreiben, deren Durchmesser geringfügig kleiner als der Durchmesser des Drahtnetzes 31 ist. Der Flansch 34 bildet somit eine Anschlagfläche für das in das Messrohr 29, 30 eingesetzte Drahtnetz 31 und begrenzt dessen Bewegung in einer axialen Richtung.

In der anderen axialen Richtung wird das Drahtnetz 31 durch das eingeschraubte Teilrohr 30 gehalten. Das Drahtnetz 31 ist somit zwischen dem Flansch 34 und einem stirnseitigen Bereich des Teilrohres 30 eingeklemmt. Die Fixierung des Drahtnetzes 31 im Messrohr 29, 30 wird folglich ausschließlich dadurch bewerkstelligt, dass das Teilrohr 30 in die Aufnahme 32 eingeschraubt wird.

Das Drahtnetz 31 kann somit problemlos aus dem Messrohr 29, 30 entnommen werden, indem lediglich die Schraubverbindung zwischen dem Teilrohr 30 und der Aufnahme 32 gelöst wird. In diesem Fall liegt das Drahtnetz 31 frei und fällt bei einem entsprechenden Verschwenken des Messkopfes aus der Aufnahme 32 heraus.

Vorteilhaft an dem beschriebenen Messkopf ist die Tatsache, dass sämtliche mit dem strömenden Gas in Verbindung stehende Teile (Teilrohr 29, Teilrohr 30, Schutzelemente 33, Drahtnetz 31) schnell und problemlos lediglich durch das Lösen zweier Schraubverbindungen, nämlich der Schraubverbindung zwischen Teilrohr 29 und der Aufnahme 32 und der Schraubverbindung zwischen Teilrohr 30 und der Aufnahme 32 vom restlichen Messkopf gelöst und ohne Schwierigkeiten gereinigt bzw. ausgewechselt werden können. Dies ist von besonderer Bedeutung, da beispielsweise beim Durchströmen des Messrohres 29, 30 mit Atemluft Verunreinigungen der mit der Atemluft in Kontakt tretenden Teile nicht zu vermeiden sind.

Um einem Feuchtigkeitsniederschlag beim Durchströmen des Messrohrs 29, 30 mit Atemluft zu vermeiden, ist das Drahtnetz 31 mit einer Wicklung 35 zur indirekten bzw. induktiven Heizung gekoppelt.

Beiderseits des Drahtnetzes 31 sind sich über den Umfang des Messrohres 29, 30 erstreckende Ringkanäle 36, 37 vorgesehen, die einerseits mit dem Innenraum des Messrohrs 29, 30 und andererseits mit zumindest einem Drucksensor 38 in Verbindung stehen. Die Drucksensoren 38 sind dabei in einer Halterung 39 gehalten und mit einer im Handgriff 28 untergebrachten Auswerteelektronik 40 gekoppelt.

Die Ringkanäle 36, 37 werden einerseits von einem radial innenliegenden Bereich der Aufnahme 32 und andererseits von einem radial außen oder stirnseitig liegenden Bereich der Teilrohre 29, 30 begrenzt. In dem in der Figur dargestellten Ausführungsbeispiel wird darüber hinaus ein Teil der Ringkanalwandungen von Dichtelementen 41 gebildet, welche in entsprechende, in der äußeren Mantelfläche der Teilrohre 29, 30 ausgebildete Ringnuten eingesetzt sind.

Somit werden die Ringkanäle 36, 37 automatisch durch das Einschrauben der Teilrohre 29, 30 in die Aufnahme 32 gebildet.

Die Ringkanäle 36 sind in dem in der Figur gezeigten Ausführungsbeispiel so angeordnet, dass ein Ringkanal 36 mit dem in Strömungsrichtung gesehen vor dem Drahtnetz 31 gelegenen Bereich und ein Ringkanal 36 mit dem in Strömungsrichtung hinter dem Drahtnetz 31 gelegenen Bereich verbunden ist. Beim Anschließen dieser beiden Ringkanäle 36 an einen Differenzdrucksensor 38 kann der am Drahtnetz 31 stattfindende Druckabfall bestimmt werden.

Ein weiterer Ringkanal 37 kann so ausgebildet werden, dass er mit der Umgebungsluft in Verbindung steht, deren momentaner Druck dann über einen entsprechenden Drucksensor 38 ermittelt werden kann, welcher mit dem Ringkanal 37 gekoppelt ist.

Bei der Verwendung des in der Figur dargestellten Messkopfes mit einem Rhinomanometrie-Aufsatz wird anstelle des Teilrohres 30 eben dieser Aufsatz in die Aufnahme 32 eingeschraubt. Der Aufsatz ist dabei so ausgebildet, dass er ebenso wie das Teilrohr 30 zur Bildung der Ringkanäle 36, 37 beiträgt.

Der in der Figur dargestellte Messkopf kann folglich sowohl für die Lungenfunktionsanalyse als auch für die Rhinomanometrie eingesetzt werden.

## Patentansprüche

1. Messkopf zur Bestimmung eines Gasvolumenstroms mit einem insbesondere an einem Handgriff (28) angeordneten Messrohr (29, 30), das mit einem sich im wesentlichen senkrecht zu seiner Achse erstreckenden feinmaschigen Drahtnetz (31) versehen ist, an dessen beiden Seiten eine Druckerfassung erfolgt, wobei das Messrohr (29, 30) aus zwei im wesentlichen koaxial ausgerichteten, mit dem Messkopf lösbar verbundenen Teilrohren (29, 30) besteht, zwischen denen das Drahtnetz (31) gehalten ist,
**dadurch gekennzeichnet,**
**dass** beiderseits des Drahtnetzes (31) sich über den Umfang des Messrohrs (29, 30) erstreckende Ringkanäle (36, 37) ausgebildet sind, die einerseits mit dem Innenraum des Messrohrs (29, 30) und andererseits mit zumindest einem Drucksensor (38) in Verbindung stehen, wobei die Ringkanäle (36, 37) zwischen den Außenwänden oder den Stirnseiten der Teilrohre (29, 30) und einer im Messkopf vorgesehenen Aufnahme (32) für die Teilrohre ausgebildet sind.

2. Messkopf nach Anspruch 1,
**dadurch gekennzeichnet,**
**dass** die Teilrohre (29, 30) in die im Messkopf vorgesehene Aufnahme (32) einschraubbar sind.

3. Messkopf nach Anspruch 1 oder 2,
**dadurch gekennzeichnet,**
**dass** beiderseits des Drahtnetzes (31) Durchbrechungen aufweisende Schutzelemente (33) vorgesehen sind.

4. Messkopf nach Anspruch 3,
**dadurch gekennzeichnet,**
**dass** sich die Schutzelemente (33) in Strömungsrichtung über einen Bereich von 0,3 bis 0,8 cm erstrecken.

5. Messkopf nach einem der Ansprüche 3 oder 4,
**dadurch gekennzeichnet,**
**dass** die Schutzelemente (33) eine Wabenstruktur aufweisen, wobei die einzelnen Waben in Strömungsrichtung ausgerichtet sind.

6. Messkopf nach Anspruch 5,
**dadurch gekennzeichnet,**
**dass** die einzelnen Waben einen Durchmesser von 3 bis 3,5 mm aufweisen.

7. Messkopf nach einem der Ansprüche 3 bis 6,
**dadurch gekennzeichnet,**
**dass** in jedem Teilrohr (29, 30) eine Einrichtung zur lösbaren Befestigung eines Schutzelements (33) vorgesehen ist und / oder dass die Schutzelemente (33) in die Teilrohre (29, 30) einschraubbar sind.

8. Messkopf nach einem der Ansprüche 1 bis 7,
**dadurch gekennzeichnet,**
**dass** die Teilrohre (29, 30) jeweils an ihrem dem Drahtnetz (31) abgewandten Endbreich mit einem düsenartigen, sich mit zunehmendem Abstand vom Drahtnetz (31) stetig verjüngenden Ansatz versehen sind, wobei insbesondere jedes Teilrohr (29, 30) in einem Bereich zwischen dem düsenartigen Ansatz und dem Schutzelement (33) bzw. dem Drahtnetz (31) im wesentlichen zylindrisch ausgebildet ist.

9. Messkopf nach einem der Ansprüche 1 bis 8,
**dadurch gekennzeichnet,**
**dass** im Bereich der Ringkanäle (36, 37) zwischen den Außenwänden oder Stirnseiten der Teilrohre (29, 30) und der im Messkopf vorgesehenen Aufnahme (32) Dichtungen (41) vorgesehen sind und/oder dass die Aufnahme (32) mit einem Gewinde insbesondere zur Kopplung mit einem Aufsatz, vorzugsweise einem Rhinomanometrie-Aufsatz versehen ist.

10. Messkopf nach einem der Ansprüche 1 bis 9,
**dadurch gekennzeichnet,**
**dass** ein Sensor (38) zur Ermittlung des Umgebungsdrucks vorgesehen ist und / oder dass das Drahtnetz (31) mit einer insbesondere regelbaren Einrichtung (35) zur Aufheizung desselben gekoppelt ist und / oder dass sämtliche mit dem Gasvolumenstrom in Kontakt tretenden Teile, insbesondere die Teilrohre (29, 30), das Drahtnetz (31) und gegebenenfalls die Schutzelemente (33) lösbar mit dem Messkopf verbunden sind.

## Claims

1. Measuring head for the determination of a volume flow of a gas with a measuring tube (29, 30) arranged on a handle (28) the measuring tube being provided with a fine mesh wire net (31) extending substantially perpendicular to its axis with a pressure determination taking place at both sides thereof, wherein the measuring tube (29, 30) consists of two at least substantially coaxially aligned part tubes (29, 30) releasably connected to the measuring head between which the wire net (31) is held,
**characterized in that**
ring channels (36, 37) extending over the periphery of the measuring tube (29, 30) are formed on both sides of the wire net (31) and communicate on the one hand with the inner space of the measuring tube (29, 30) and on the other hand with at least one pressure sensor (38), with the ring channels (36, 37) being formed between the outer walls or the end faces of the part tubes (29, 30) and a mount (32) for the part tubes provided in the measuring head.

2. Measuring head in accordance with claim 1,
**characterized in that**
the part tubes (29, 30) can be screwed into the mount (32) provided in the measuring head.

3. Measuring head in accordance with claim 1 or claim 2,
**characterized in that**
protective elements (33) having apertures are provided on both sides of the wire net (31).

4. Measuring head in accordance with claim 3,
**characterized in that**
the protective elements (33) extend in the flow direction over a range of 0.3 to 0.8 cm.

5. Measuring head in accordance with one of the claims 3 or 4,
**characterized in that**
the protective elements (33) have a honeycomb-structure with the individual cells being aligned in the flow direction.

6. Measuring head in accordance with claim 5,
**characterized in that**
the individual cells have a diameter of 3 to 3.5 mm.

7. Measuring head in accordance with one of the claims 3 to 6,
**characterized in that**
a device is provided in each part tube (29, 30) for the releasable securing of a protective element (33) and/or **in that** the protective elements (33) can be screwed into the part tubes (29, 38).

8. Measuring head in accordance with one of the claims 1 to 7,
**characterized in that**
the part tubes (29, 30) are each provided at their end region remote from the wire net (31) with a nozzle-like formation continuously tapering with increasing distance from the wire net (31), with each part tube (29, 30) in particular being made substantially cylindrical in a region between the nozzle-like formation and the protective elements (33) and/or the wire net (31).

9. Measuring head in accordance with one of the claims 1 to 8,
**characterized in that**
seals (41) are provided in the region of the ring channels (36, 37) between the outer walls or end sides of the part tubes (29, 30) and the mount (32) provided in the measuring head and/or **in that** the mount (32) is provided with a thread, in particular for coupling with a fixture, preferably a rhinomanometry fixture.

10. Measuring head in accordance with one of the claims 1 to 9,
**characterized in that**
a sensor (38) is provided for determining the environmental pressure and/or **in that** the wire net (31) is provided with an in particular regulatable device (35) for the heating of the same and/or **in that** all parts which enter into contact with the volume flow of gas, in particular the part tubes (29, 30), the wire net (31) and optionally the protective elements (33) are releasably connected to the measuring head.

## Revendications

1. Tête de mesure pour déterminer un courant volumique de gaz, comportant un tube de mesure (29, 30) agencé en particulier sur une poignée à main (28) et pourvu d'une grille de fils à mailles fines (31) qui s'étend sensiblement perpendiculairement à son axe et sur les deux côtés de laquelle s'effectue une détection de pression, dans laquelle le tube de mesure (29, 30) est constitué par deux tubes partiels (29, 30) orientés sensiblement coaxialement et reliés de façon détachable à la tête de mesure, entre lesquels est maintenue la grille de fils (31),
**caractérisée en ce qu'**il est prévu des canaux annulaires (36, 37) qui s'étendent des deux côtés de la grille de fils (31) sur la périphérie du tube de mesure (29, 30) et qui sont reliés d'une part au volume intérieur du tube de mesure (29, 30) et d'autre part à au moins un détecteur de pression (38), les canaux annulaires (36, 37) étant réalisés entre les parois extérieures ou les faces frontales des tubes partiels (29, 30) et un logement (32) prévu dans la tête de mesure pour les tubes partiels.

2. Tête de mesure selon la revendication 1, **caractérisée en ce que** les tubes partiels (29, 30) peuvent être vissés dans le logement (32) prévu dans la tête de mesure.

3. Tête de mesure selon l'une ou l'autre des revendications 1 et 2, **caractérisée en ce que** des éléments de protection (33) présentant des traversées sont prévus des deux côtés de la grille de fils (31).

4. Tête de mesure selon la revendication 3, **caractérisée en ce que** les éléments de protection (33) s'étendent en direction d'écoulement sur une zone de 0,3 à 0,8 cm.

5. Tête de mesure selon l'une ou l'autre des revendications 3 et 4, **caractérisée en ce que** les éléments de protection (33) présentent une structure en nids d'abeilles, les nids individuels étant orientés en direction d'écoulement.

6. Tête de mesure selon la revendication 5, **caractérisée en ce que** les nids individuels présentent un diamètre de 3 à 3,5 mm.

7. Tête de mesure selon l'une des revendications 3 à 6, **caractérisée en ce qu'**il est prévu dans chaque tube partiel (29, 30) un dispositif pour la fixation détachable d'un élément de protection (33), et/ou **en ce que** les éléments de protection (33) peuvent être vissés dans les tubes partiels (29, 30).

8. Tête de mesure selon l'une des revendications 1 à 7, **caractérisée en ce que** les tubes partiels (29, 30) sont pourvus, chacun dans leur zone d'extrémité détournée de la grille de fils (31), d'un talon en forme de buse qui va en se rétrécissant constamment avec l'augmentation de la distance depuis la grille de fils (31), en particulier chaque tube partiel (29, 30) étant réalisé sensiblement cylindrique dans une zone entre le talon en forme de buse et l'élément de protection (33) ou la grille de fils (31).

9. Tête de mesure selon l'une des revendications 1 à 8, **caractérisée en ce qu'**il est prévu des joints d'étanchement (41) dans la zone des canaux annulaires (36, 37) entre les parois extérieures ou les faces frontales des tubes partielles (29, 30) et le logement (32) prévu dans la tête de mesure, et/ou **en ce que** le logement (32) est pourvu d'un pas de vis en particulier pour le couplage avec un accessoire de connexion, de préférence un accessoire de connexion de rhinomanométrie.

10. Tête de mesure selon l'une des revendications 1 à 9, **caractérisée en ce qu'**il est prévu un détecteur (38) pour déterminer la pression environnante et/ou **en ce que** la grille de fils (31) est couplée à un dispositif (35), en particulier réglable, pour la chauffer, et/ou **en ce que** toutes les pièces venant en contact avec le courant volumique de gaz, en particulier les tubes partiels (29, 30), la grille de fils (31) et le cas échéant les éléments de protection (33) sont reliés de façon détachable à la tête de mesure.
